(19) Europäisches Patentamt
European Patent Office
Office européen des brevets

(11) EP 2 007 771 B1

(12) **EUROPEAN PATENT SPECIFICATION**

(45) Date of publication and mention of the grant of the patent:
**07.04.2010 Bulletin 2010/14**

(21) Application number: **07728054.3**

(22) Date of filing: **12.04.2007**

(51) Int Cl.:
***C07D 498/04*** *(2006.01)*

(86) International application number:
**PCT/EP2007/053587**

(87) International publication number:
**WO 2007/116101 (18.10.2007 Gazette 2007/42)**

(54) **IMIDAZO COMPOUNDS**

IMIDAZO-VERBINDUNGEN

COMPOSÉS IMIDAZO

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.04.2006 CH 62006**

(43) Date of publication of application:
**31.12.2008 Bulletin 2009/01**

(73) Proprietor: **Novartis AG**
**4056 Basel (CH)**

(72) Inventors:
- **HEROLD, Peter**
  **4142 Münchenstein (CH)**
- **MAH, Robert**
  **4132 Muttenz (CH)**
- **TSCHINKE, Vincenzo**
  **4102 Binningen (CH)**
- **STOJANOVIC, Aleksandar**
  **4055 Basel (CH)**
- **MARTI, Christiane**
  **4310 Rheinfelden (CH)**
- **JELAKOVIC, Stjepan**
  **79100 Freiburg (CH)**
- **STUTZ, Stefan**
  **4053 Basel (CH)**

(74) Representative: **Warner, James Alexander et al**
**Carpmaels & Ransford**
**43-45 Bloomsbury Square**
**London WC1A 2RA (GB)**

(56) References cited:
**WO-A-2006/005726**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 007 771 B1

## Description

FIELD OF THE INVENTION

The invention relates to novel heterocyclic compounds, to a process for preparing the compounds, to pharmaceutical products containing them, and to their use as active pharmaceutical ingredients, in particular as aromatase inhibitors.

DETAILED DESCRIPTION OF THE INVENTION

The present invention relates firstly to compounds of the general formula

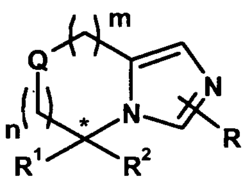

(I) ,

in which

- R is deuterium, halogen or hydrogen;
- $R^1$ is aryl-$C_0$-$C_4$-alkyl or heterocyclyl-$C_0$-$C_4$-alkyl, which radicals may be substituted by 1-4 $C_1$-$C_8$ alkoxy, $C_1$-$C_8$ alkoxycarbonyl, $C_1$-$C_8$ alkyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, oxide, oxo, tri-$C_1$-$C_4$-alkylsilyl, trifluoromethoxy or trifluoromethyl;
- $R^2$ is

  a) deuterium, halogen, hydroxy, cyano or hydrogen; or
  b) $C_2$-$C_8$ alkenyl, $C_2$-$C_8$ alkynyl, $C_1$-$C_8$ alkoxy, $C_1$-$C_4$ alkoxycarbonyl-$C_1$-$C_4$ alkyl, $C_1$-$C_8$ alkyl, $C_0$-$C_4$ alkylcarbonyl, aryl-$C_o$-$C_4$ alkyl, carboxy-$C_1$-$C_4$ alkyl, $C_3$-$C_8$ cycloalkyl or heterocyclyl-$C_0$-$C_4$ alkyl, which radicals may be substituted by 1-4 $C_1$-$C_8$ alkoxy, $C_1$-$C_8$ alkoxycarbonyl, $C_1$-$C_8$ alkyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, oxide, oxo, tri-$C_1$-$C_4$ alkylsilyl, trifluoromethoxy or trifluoromethyl;

- Q is oxygen or sulphur;
- m is a number 0, 1 or 2;
- n is a number 0, 1 or 2; and
- * designates an asymmetric carbon atom; and

where

m and n are not simultaneously 0;
and salts, preferably pharmaceutically acceptable salts, thereof.

A compound of formula (I) is to be understood as a compound having a specific configuration around the designated asymmetric carbon atom labelled "*". If a synthesis method is used which leads to racemic compounds, the racemate resolution is carried out in accordance with conventional methods, such as via a chiral HPLC column. Compounds of the formula (I) as described in the present invention exhibit a pronounced aromatase inhibitory activity. The aforementioned activity can, readily and as described below, be determined by using a commercial Cyp19 enzyme inhibition kit, preferably the Cyp19/methoxy-4-trifluoromethyl-coumarin (MFC) high throughput inhibition kit (Becton Dickinson Biosciences, San Jose, CA, USA) as described hereafter. In the above-mentioned inhibition kit, compounds of the formula (I) show an inhibiting activity which is at least 10 times higher, but preferably 20 times higher, or more preferably 40 times higher, than the substances of the formula (I) with the opposite configuration around the asymmetric carbon atom labelled "*". A higher inhibiting activity corresponds to a lower $IC_{50}$ value.
The term aryl stands for a mono-, bi- or tricyclic aromatic hydrocarbon complying with the Hückel rule which

generally comprises 6-14, preferably 6-10, carbon atoms and is for example phenyl, naphthyl, e.g. 1- or 2-naphthyl or anthracenyl. Aryl having 6-10 carbon atoms, in particular phenyl or 1- or 2-naphthyl, is preferred. The stated radicals may be unsubstituted or substituted one or more times, e.g. once or twice, in which case the substituent may be in any position, e.g. in the o, m or p position of the phenyl radical or in the 3 or 4 position of the 1- or 2-naphthyl radical, and there may also be a plurality of identical or different substituents present. Examples of substituents on aryl radicals or the preferred phenyl or naphthyl radicals are: $C_1$-$C_8$ alkoxy, $C_1$-$C_8$ alkoxycarbonyl, $C_1$-$C_8$ alkyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, tri-$C_1$-$C_4$ alkylsilyl, trifluoromethoxy or trifluoromethyl.

Aryl-$C_0$-$C_4$ alkyl is for example phenyl, naphthyl or benzyl.

The term heterocyclyl stands for a saturated, partially saturated or unsaturated, 4-8-membered, particularly preferably 5-membered, monocyclic ring system, for a saturated, partially saturated or unsaturated, 7-12-membered, particularly preferably 9-10-membered, bicyclic ring system and also for a partially saturated or unsaturated, 9-12-membered tricyclic ring system which comprises an N, O or S atom in at least one of the rings, it being possible for an additional N, O or S atom to be present in one ring. Said radicals may be unsubstituted or substituted one or more times, e.g. once or twice, and there may also be a plurality of identical or different substituents present. Examples of substituents on heterocyclyl radicals are: $C_1$-$C_8$ alkoxy, $C_1$-$C_8$ alkoxycarbonyl, $C_1$-$C_8$ alkyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, oxide, oxo, tri-$C_1$-$C_4$ alkylsilyl, trifluoromethoxy or trifluoromethyl.

Saturated heterocyclyl-$C_0$-$C_4$ alkyl is for example azepanyl, azetidinyl, aziridinyl, 3,4-dihydroxypyrrolidinyl, 2,6-dimethylmorpholinyl, 3,5-dimethylmorpholinyl, dioxanyl, [1,4]dioxepanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, 4-methylpiperazinyl, 1-methylpiperidinyl, 1-methylpyrrolidinyl, morpholinyl, oxathianyl, oxepanyl, 2-oxo-azepanyl, 2-oxo-imidazolidinyl, 2-oxo-oxazolidinyl, 2-oxo-piperidinyl, 4-oxo-piperidinyl, 2-oxo-pyrrolidinyl, 2-oxo-tetrahydropyrimidinyl, 4-oxo-thiomorpholinyl, piperazinyl, piperidinyl, pyrrolidinyl, tetrahydrofuranyl, tetrahydropyranyl, tetrahydrothiophenyl, tetrahydrothiopyranyl, thiepanyl or thiomorpholinyl.

Partially saturated bicyclic heterocyclyl-$C_0$-$C_4$ alkyl is for example 3,4-dihydro-2H-benzo[1,4]oxazinyl, 4,5,6,7-tetrahydrobenzofuranyl or 4,5,6,7-tetrahydrobenzothiazolyl.

Unsaturated bicyclic heterocyclyl-$C_0$-$C_4$ alkyl is for example benzofuranyl, benzoimidazolyl, benzo[d]isothiazolyl, benzo[d]isoxazolyl, benzo[b]thiophen-yl, quinolinyl, imidazo[1,5-a]pyridinyl, indazolyl, indolyl or isoquinolinyl.

Unsaturated monocyclic heterocyclyl-$C_0$-$C_4$ alkyl is for example imidazolyl, oxazolyl, pyridyl, pyrrolyl, tetrazolyl, thiazolyl or thiophenyl.

$C_2$-$C_8$ alkenyl is for example ethenyl, propenyl, isopropenyl, butenyl, isobutenyl, secondary butenyl, tertiary butenyl, or a pentenyl, hexenyl or heptenyl group.

$C_2$-$C_8$ alkynyl is for example ethynyl, propynyl, butynyl, or a pentynyl, hexynyl or heptynyl group.

$C_1$-$C_8$ alkoxy is for example $C_1$-$C_5$ alkoxy such as methoxy, ethoxy, propoxy, isopropoxy, butoxy, isobutoxy, secondary butoxy, tertiary butoxy or pentoxy, but may also be a hexoxy or heptoxy group.

$C_1$-$C_8$ alkoxycarbonyl is preferably $C_1$-$C_4$ alkoxycarbonyl such as methoxycarbonyl, ethoxycarbonyl, propoxycarbonyl, isopropoxycarbonyl, butoxycarbonyl, isobutoxycarbonyl, secondary butoxycarbonyl or tertiary butoxycarbonyl.

$C_1$-$C_4$ alkoxycarbonyl-$C_1$-$C_4$ alkyl is for example methoxycarbonylmethyl or ethoxycarbonylmethyl, 2-methoxycarbonylethyl or 2-ethoxycarbonylethyl, 3-methoxycarbonylpropyl or 3-ethoxycarbonylpropyl or 4-ethoxycarbonylbutyl.

$C_1$-$C_8$ alkyl may be straight-chain or branched and/or bridged and is for example methyl, ethyl, propyl, isopropyl, butyl, isobutyl, secondary butyl, tertiary butyl, or a pentyl, hexyl or heptyl group.

$C_0$-$C_8$ alkylcarbonyl is for example formyl, acetyl, propionyl, propylcarbonyl, isopropylcarbonyl, butylcarbonyl, isobutylcarbonyl, secondary butylcarbonyl or tertiary butylcarbonyl.

Carboxy-$C_1$-$C_4$ alkyl is for example carboxymethyl, 2-carboxyethyl, 2- or 3-carboxypropyl, 2-carboxy-2-methylpropyl, 2-carboxy-2-ethylbutyl, or 4-carboxybutyl, in particular carboxymethyl.

$C_3$-$C_8$ cycloalkyl is preferably 3-, 5- or 6-membered cycloalkyl, such as cyclopropyl, cyclopentyl, cyclohexyl.

Halogen is for example fluorine, chlorine, bromine or iodine.

The compound groups mentioned below are not to be regarded as closed; on the contrary, parts of these compound groups may be replaced by one another or by the definitions given above, or be omitted, in a meaningful way, e.g. to replace general by more specific definitions. The definitions mentioned apply within the scope of general chemical principles such as, for example, the usual valencies of atoms.

R is preferably deuterium or hydrogen.

$R^1$ is preferably aryl, very particularly preferably mono-, di- or tri-substituted phenyl, or heterocyclyl, very particularly preferably optionally mono-, di- or tri-substituted benzofuranyl, benzo[b]thiophenyl, benzoimidazolyl, benzo[d]isothiazolyl, benzo[d]isoxazolyl, benzo[b]thiophenyl, imidazolyl, indazolyl, indolyl, oxazolyl, pyridyl, pyrrolyl, thiazolyl or thiophenyl.

$R^2$ is preferably $C_1$-$C_8$ alkoxy, hydroxy, $C_1$-$C_8$ alkyl, aryl-$C_0$-$C_4$ alkyl, deuterium, halogen, cyano or hydrogen.

Q is preferably oxygen.

n is preferably a number 0 or 1. n is particularly preferably the number 1.

m is particularly preferably the number 1.

Preferred substituents for aryl or heterocyclyl are $C_1$-$C_8$ alkoxy, $C_1$-$C_8$ alkyl, $C_1$-$C_8$ alkylcarbonyl, $C_1$-$C_8$ alkylsulphonyl, optionally substituted aryl, cyano, halogen, optionally substituted heterocyclyl, nitro, oxide, trifluoromethyl, trifluoromethoxy or trimethylsilanyl. Very particularly preferred substituents for aryl or heterocyclyl are acetyl, bromine, chlorine, cyano, fluorine, methanesulphonyl, methoxy, nitro, oxazolyl, oxide, optionally substituted phenyl, optionally substituted tetrazolyl, optionally substituted thiazolyl or optionally substituted thiophenyl.

It is likewise preferred for $R^1$ to be a mono-, di - or tri-substituted unsaturated heterocyclyl substituent, where the substituents are preferably selected from the group consisting of $C_1$-$C_8$ alkyl, $C_1$-$C_8$ alkoxy, $C_1$-$C_8$ alkoxycarbonyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, oxide, oxo, tri-$C_1$-$C_4$ alkylsilyl, trifluoromethoxy and trifluoromethyl.

Compounds having a second asymmetric carbon atom can exist in the form of optically pure diastereomers, mixtures of diastereomers, diastereomeric racemates, mixtures of diastereomeric racemates, or meso compounds. The invention embraces all these forms.

Mixtures of, diastereomers, diastereomeric racemates, or mixtures of diastereomeric racemates can be fractionated by conventional methods, such as by racemate resolution, column chromatography, thin-layer chromatography, HPLC and the like.

The expression "pharmaceutically acceptable salts" embraces salts with organic or inorganic acids, such as hydrochloric acid, hydrobromic acid, nitric acid, sulphuric acid, phosphoric acid, citric acid, formic acid, maleic acid, acetic acid, succinic acid, tartaric acid, methanesulphonic acid, p-toluenesulphonic acid and the like. Salts of compounds containing salt-forming groups are, in particular, acid addition salts, salts with bases or else, if appropriate, if two or more salt-forming groups are present, are mixed salts or inner salts.

The compounds of the formula (I) can be prepared in an analogous manner to the preparation processes disclosed per se in the literature (1H-imidazol-4-yl)methanol by conversion into methyl (1H-imidazol-4-ylmethoxy) acetate followed by a Grignard addition, subsequent reduction (or vice versa) and ring closure, followed by separation into the antipodes with regard to the carbon atom labelled "*". (Scheme I).

Scheme I:

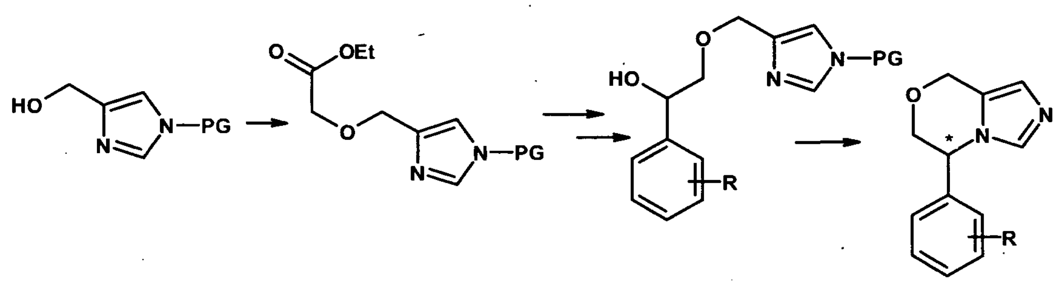

Alternatively, the compounds of the formula (I) can be obtained in an analogous manner to the preparation processes disclosed per se in the literature starting from hydroxyphenylacetic acid derivatives by reaction with (1H-imidazol-4-yl)methanols followed by a reduction and subsequent ring closure, followed by separation into the antipodes with regard to the carbon atom labelled "*". (Scheme II).

Scheme II:

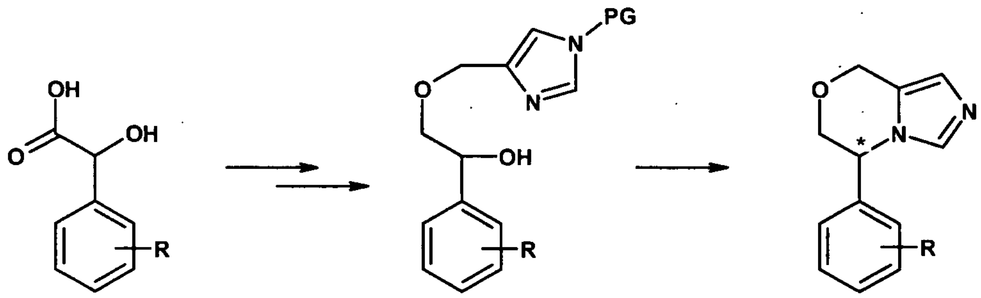

Details of the specific preparation variants can be found in the examples.

Separation into antipodes is possible by methods known per se, either, preferably, at an early stage in synthesis, by salt formation with an optically active acid such as, for example, (+)- or (-)-mandelic acid and separation of the diastereomeric salts by fractional crystallization, or, preferably, at a fairly late stage, by derivatization with a chiral auxiliary component, such as, for example, (+)- or (-)-camphanyl chloride and separation of the diastereomeric products by chromatography and/or crystallization and subsequent cleavage of the bond to the chiral auxiliary. The pure diastereomeric salts and derivatives can be analysed to determine the absolute configuration of the compound present, using customary spectroscopic methods, with single-crystal X-ray spectroscopy representing one particularly appropriate method.

Salts are primarily the pharmaceutically acceptable or non-toxic salts of compounds of the formula (I). Such salts are formed for example by compounds of the formula (I) containing an acidic group, such as a carboxyl or sulpho group and are, for example, salts thereof with suitable bases, such as non-toxic metal salts derived from metals of group Ia, Ib, IIa and IIb of the Periodic Table of the Elements, such as alkali metal salts, especially lithium, sodium or potassium salts, alkaline earth metal salts, magnesium or calcium salts for example, and also zinc salts or ammonium salts, and additionally salts formed with organic amines, such as unsubstituted or hydroxyl-substituted mono-, di- or trialkylamines, especially mono-, di- or tri-lower alkylamines, or with quaternary ammonium bases, e.g. methyl-, ethyl-, diethyl- or triethylamine, mono-, bis- or tris(2-hydroxyl-lower alkyl)amines, such as ethanolamine, diethanolamine or triethanolamine, tris(hydroxylmethyl)methylamine or 2-hydroxyl-tertiary-butylamine, N,N-di-lower alkyl-N-(hydroxyl-lower alkyl)amine, such as N,N-di-N-dimethyl-N-(2-hydroxylethyl)amine, or N-methyl-D-glucamine, or quaternary ammonium hydroxides, such as tetrabutylammonium hydroxide. The compounds of the formula (I) containing a basic group, such as an amino group, can form acid addition salts, with suitable inorganic acids for example, such as hydrohalic acid, such as hydrochloric acid, hydrobromic acid, or sulphuric acid with replacement of one or both protons, phosphoric acid with replacement of one or more protons, orthophosphoric acid or metaphosphoric acid for example, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxylmaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, such as the α-amino acids specified earlier on, and also methanesulphonic acid, ethanesulphonic acid, 2-hydroxylethanesulphonic acid, ethane-1,2-disulphonic acid, benzenesulphonic acid, 4-toluenesulphonic acid, naphthalene-2-sulphonic acid, 2- or 3-phospho-glycerate, glucose 6-phosphate, N-cyclohexylsulphamic acid (to form cyclamates), or with other acidic organic compounds, such as ascorbic acid. Compounds of the formula (I) containing acidic and basic groups can also form inner salts.

Isolation and purification can also be carried out using pharmaceutically unsuitable salts.

The compounds of the formula (I) also include those compounds in which one or more atoms have been replaced by their stable, non-radioactive isotopes: for example, a hydrogen atom by deuterium.

Prodrug derivatives of the presently described compounds are derivatives thereof which when employed *in vivo* release the original compound as a result of a chemical or physiological process. A prodrug may be converted into the original compound, for example, when a physiological pH is reached or as a result of enzymatic conversion. Examples of possible prodrug derivatives include esters of freely available carboxylic acids, S- and O-acyl derivatives of thiols, alcohols or phenols, the acyl group being defined as above. Preference is given to pharmaceutically useful ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters, such as lower ω-(amino, mono- or dialkylamino, carboxyl, lower alkoxycarbonyl)-alkyl esters or such as lower α-(alkanoyloXy,

alkoxycarbonyl or dialkylaminocarbonyl)alkyl esters; pivaloyloxymethyl esters and similar esters are conventionally used as ester derivatives of this kind.

Because of the close relationship between a free compound, a prodrug derivative and a salt compound, a defined compound in this invention also includes its prodrug derivative and salt form, insofar as this is possible and appropriate.

The naturally occuring estrogens 17β-estradiol (E2), estrone (E1) and estriol (E3) are C18 steroids derived from cholesterol. After binding to lipoprotein receptors, cholesterol is taken up by steroidogenic cells, stored and moved to the sites of steroid synthesis. Aromatization of the A-ring in the steroid scaffold is the last step in the formation of estrogen. This reaction is catalyzed by the P450 aromatase monooxygenase enzyme complex (Cyp19) that is present in the smooth endoplasmic reticulum and functions as a demethylase. In three consecutive hydroxylating reactions, estrone and estradiol are formed from their obligatory precursors androstenedione and testosterone, respectively.

The primary sources of estradiol in woman are the theca and granulose cells of the ovaries and the luteinized derivatives of these cells. According to the "two-cell" theory of estrogen synthesis, the theca cells secrete androgens that diffuse to the granulose cells to be aromatized to estrogens. There is, however, evidence that both cell types are enabled to form both androgens and estrogens. Estrone and estriol are primarily formed in the liver from estradiol. Aromatase activity has also been detected in muscle, fat , nervous tissue and the Leydig cells of the testes. The level of estrogen synthesis in extragonadal tissues increases as a function of age and body weight.

In the serum, estradiol reversibly binds to sex-hormone-binding globulin, a β-globulin, and with lesser affinity to albumin; about 2-3 percent is unbound. Estrogens are metabolized by sulfation or glucuronidation, and the conjugates are excreted into the bile or urine. Hydrolysis of these conjugates by the intestinal flora and subsequent reabsorption of the estrogens results in enterohepatic circulation.

Estrogens stimulate growth, blood flow and water retention in sexual organs and are also involved in causing breast cancer and endometrial tumors. In the liver, estrogens increase the expression of lipoprotein receptors that results in a decrease in serum concentrations of low-density lipoprotein cholesterol. Estrogens also increase the potential for coagulation by stimulating the production of coagulation factors in the liver. In bone, both osteoclasts and osteoblasts are direct targets of estrogens, but overall, estrogens are classified as antiresorptive agents.

In breast tissue, estrogens stimulate the growth and differentiation of the ductal epithelium, induce mitotic activity of ductal cylindric cells and stimulate the growth of connective tissue. Estrogens stimulate the growth of breast cancer cells. In postmenopausal women with breast cancer, the tumor concentration of estradiol is high caused by *in situ* aromatization, despite the presence of low serum estradiol concentrations.

The compounds described in the present invention have useful pharmacological properties as they selectively inhibit the enzyme aromatase (Cyp19) in mammals, including humans. As a result, the metabolic conversion of androgens into estrogens is inhibited. The compounds are therefore suitable, for example, for the treatment of estrogen-dependent diseases, including estrogen-dependent breast cancer, particularly in postmenopausal women. They are also useful, for example, in the treatment of gynaecomastia, that is to say the development of breasts in men, as the aromatization of steroids can be inhibited by the described compounds.

These effects are demonstrable in *in vitro* assay tests using cell-free and cellular systems. The *in vitro* inhibition of aromatase activity of the compounds of the present invention can be demonstrated by using a commercial Cyp19 enzyme inhibition kit. The Cyp19/Methoxy-4-trifluoromethyl-coumarin (MFC) high throughput inhibition kit (Becton Dickinson Biosciences, San Jose, CA, USA), for example, is designed to screen for potential inhibitors of Cyp19 catalytic activity in a 96-well format. The kit includes recombinant human Cyp19 enzyme in the form of supersomes, a fluorescent P450 substrate, an NADPH regenerating system, a reaction buffer and a stop reagent. MFC, the fluorogenic substrate is rapidly converted by Cyp19 supersomes to the highly fluorescent product 7-hydroxy-4-trifluoromethyl coumarin (7-HFC). The execution of the assay in the presence of various concentrations of inhibitor compounds ranging from 0.2 nanomolar to 20 millimolar occurs according to the manufacturer's instructions.

The inhibition curve is generated by fitting a 4-parameter logistic function to the raw data of the samples using the least squares approach. The function is described as follows:

$$\dot{Y} = (d-a) / ((1 + (x/c)^{-b}) + a)$$

with:

a = minimum data values
b = slope
c= $IC_{50}$
d = maximum data values

x = inhibitor concentrations

The compounds described in the present invention show Cyp19 inhibitory properties at minimal concentrations between $10^{-3}$ to $10^{-10}$ mol/l.

Example of CYP19 inhibition:

| Example number | IC50 value [nM] |
|---|---|
| 1 | 4.8 |
| antipode of 1 | 8346.3 |

The Cyp19 inhibitory properties of compounds described in the present invention can also be demonstrated in a cellular assay. The NCI-H295R human adrenocortical carcinoma cell line has been characterized in detail in the literature and shown to express most of the key enzymes necessary for steroidogenesis. These include Cyp11A (cholesterol side-chain cleavage), Cyp11B1 (steroid 11β-hydroxylase), Cyp11B2 (aldosterone synthetase), Cyp17 (steroid 17α-hydroxylase and/or 17,20 lyase), Cyp19 (aromatase), Cyp21B2 (steroid 21-hydroxylase) and 3β-HSD (hydroxysteroid dehydrogenase). The cells have the physiological characteristics of zonally undifferentiated human fetal adrenal cells, with the ability to produce the steroid hormones of each of the three phenotypically distinct zones found in the adult adrenal cortex.

The NCI-H295R cells (American Type Culture Collection, ATCC, Rockville, MD, USA) are cultured in Dulbecco's Modified Eagle'Ham F-12 medium (DME/F12) that is supplemented with Ultroser SF serum (Soprachem, Cergy-Saint-Christophe, France) as well as insulin, transferrin, selenit (I-T-S, Becton Dickinson Biosiences, Franklin Lakes, NJ, USA) and antibiotics in 75 $cm^2$ cell culture flasks at a temperature of 37°C and a 95% air/5% $CO_2$ humidified atmosphere. The cells are subsequently transferred in a 24-well plate and seeded in presence of DME/F12 medium that is supplemented with 0.1% bovine serum albumin instead of Ultroser SF serum. The experiment is initiated by incubating the cells for 72 hours in DME/F12 medium supplemented with 0.1% bovine serum albumin and test compounds in the presence or absence of cell stimulatory agents. The test compound is added in a concentration range of 0.2 nanomolar to 20 millimolar. As cell-stimulatory agents, angiotensin-II (at 10 or 100 nanomolar concentration), potassium ions (at 16 millimolar), forskolin (at 10 micromolar) or a combination of two agents are used. The cellular secretion of estrone, estradiol, dihydroepiandrostendione, aldosterone, corticosterone and/or cortisol into the cell culture medium can be quantitatively assessed with commercially available immuno-assays and specific monoclonal antibodies according to the manufacturer's instructions. The degree of secretion of a selective steroid is used as a measure of enzyme activity, respectively enzyme inhibition in the presence of absence of a test compound. The dose-dependent enzyme inhibitory activity of a compound is reflected in a inhibition curve that is characterized by an $IC_{50}$ value.

The inhibition curve is generated by fitting a 4-parameter logistic function to the raw data of the samples using the least squares approach. The function is described as follows:

$$Y = (d-a) / ((1 + (x/c)^{-b}) + a)$$

with:

a = minimum data values
b = slope
c= $IC_{50}$
d = maximum data values
x = inhibitor concentrations

The compounds described in the present invention show Cyp19 inhibitory properties at minimal concentrations between $10^{-3}$ to $10^{-10}$ mol/l.

The aromatase inhibitory effects of described compounds can be also demonstrated *in vivo* using advantageously mammalian animal models such as e.g. guinea pigs, mice, rats, cats, dogs, or monkeys.

The compound-mediated *in vivo* inhibition of aromatase activity can be tested by monitoring plasma steroid level changes as described in the following protocol: cycling female rats are injected subcutaneously 5-times on alternate days with 100 IU of pregnant mare's serum gonadotropin (PMSG, Sigma) in 0.1 ml sterile saline. Twenty-four hours after the last injection, the animals are treated orally with test compound at doses ranging from 0.01 to 10 mg/kg. Twenty-four hours after treatment, the animals are subjected to a terminal bleed. Heparinized plasma is stored at -20°C until analysis. Plasma levels of steroid (17beta-estradiol, estrone, estriol, progesterone, testosterone, aldosterone and corticosterone) are determined by commercially available radioimmunoassay kits, according to the manufacturer's instructions. A purification and concentration step is needed to measure plasma testosterone in female rats: four volumes of diethyl ether are added to the samples, mixed by gentle inversion for 15 minutes and then centrifuged for 5 minutes at 2000 rpm. The aqueous phase is frozen in dry ice and the organic phase is recovered and evaporated to dryness under a nitrogen stream. The dried extract is reconstituted in the assay buffer.

The compound-mediated *in vivo* inhibition of aromatase activity can be tested by monitoring the ovary estrogen content as follows: twenty-one day old female rats are injected subcutaneously with 10 IU pregnant mare serum gonadotropin (PMSG, Sigma). Two days later, the same rats are injected subcutaneously with 30 IU human chorionic gonadotropin (hCG, Sigma). On the day following the hCG treatment, the rats are injected subcutaneously with either propylene glycol (0.2 ml) or with various doses of the test compound. One hour later, all the rats are treated with 2.25 mg 4-androstene-3,17-dione in 0.1 ml oil, subcutaneously. Four hours after the injection of androstenedione, the rats are killed and their ovaries removed and trimmed free of adhering tissue and stored in pairs at -50°C. To determine the total estrogen content of the ovaries, 1.5 ml of 0.05 M aqueous potassium phosphate buffer (pH 7.4) and 0.2 ml of 0.1 N aqueous NaOH are added to the tissues which are then homogenized. The homogenate is extracted with 15 ml of diethyl ether - 5 ml aliquots are radioimmunoassayed with antiserum having 100% cross-reactivity with estrone, estradiol and estriol. The results are expressed as ng estrogen/pair of ovaries.

The anti-tumor activity, especially in estrogen-dependent tumors, can be demonstrated *in vivo* e.g. in dimethylbenzanthracene (DMBA)-induced mammary, tumors in female Sprague-Dawley rats (see Proc. Soc. Exp. Biol. Med. 160, 296-301, 1979). Compounds of the invention cause regression of existing tumors and suppress the appearance of new tumors at daily doses of about 1 to about 20 mg/kg p.o or less.

In order to achieve the desired effects in a patient to be treated, the compounds of the present invention can be administered orally or enterally, such as, for example, intravenously, intraperitoneally, intramuscularly, rectally, subcutaneously or else by direct injection of the active substance locally into tissues or tumours. The term patient encompasses warm-blooded species and mammals such as, for example, human, primate, bovine, dog, cat, horse, sheep, mouse, rat and pig. The compounds can be administered as pharmaceutical product or be incorporated into an administration device which ensures sustained release of the compound. The amount of substance to be administered can vary over a wide range and represent every effective dose. Depending on the patient to be treated or the condition to be treated and mode of administration, the dose of the effective substance each day can be between about 0.005 and 50 milligrams per kilogram of body weight, but is preferably between about 0.05 and 5 milligrams per kilogram of body weight each day.

For oral administration, the compounds can be formulated in solid or liquid pharmaceutical forms such as, for example, as capsules, pills, tablets, coated tablets, granules, powders, solutions, suspensions or emulsions. The dose of a solid pharmaceutical form can be one usual hard gelatine capsule which may be filled with active ingredients and excipients such as lubricants and fillers, such as, for example, lactose, sucrose and maize starch. Another form of administration may be represented by tableting of the active substance of the present invention. The tableting can take place with conventional tableting excipients such as, for example, lactose, sucrose, maize starch, combined with binder from gum acacia, maize starch or gelatine, disintegrants such as potato starch or crosslinked polyvinylpyrrolidone (PVPP) and lubricants such as stearic acid or magnesium stearate.

Examples of excipients suitable for soft gelatine capsules are vegetable oils, waxes, fats, semisolid and liquid polyols etc.

Examples of excipients suitable for producing solutions and syrups are water, polyols, sucrose, invert sugar, glucose etc.

For rectal administration, the compounds can be formulated in solid or liquid pharmaceutical forms such as, for example, suppositories. Examples of excipients suitable for suppositories are natural or hardened oils, waxes, fats, semiliquid or liquid polyols etc.

For parenteral administration, the compounds can be formulated as injectable dosage of the active ingredient in a liquid or suspension. The preparations usually comprise a physiologically tolerated sterile solvent which may comprise a water-in-oil emulsion, with or without surfactant, and other pharmaceutically acceptable excipients. Oils which can be used for such preparations are paraffins and triglycerides of vegetable, animal or synthetic origin, such as, for example, peanut oil, soya oil and mineral oil. Injectable solutions generally comprise liquid carriers such as, preferably, water, saline, dextrose or related sugar solutions, ethanol and glycols such as propylene glycol or polyethylene glycol.

The substances may be administered as transdermal patch system, as depot injection or implant if the formulation makes sustained delivery of the active ingredient possible. The active substance can be compressed as granules or to narrow cylinders and be administered subcutaneously or intramuscularly as depot injection or implant.

The pharmaceutical products may in addition also comprise preservatives, solubilizers, viscosity-increasing substances, stabilizers, wetting agents, emulsifiers, sweeteners, colorants, aromatizing agents, salts to change the

osmotic pressure, buffers, coating agents or antioxidants. They may also comprise other therapeutically valuable substances too.

The present invention further provides the use of the compounds of the formula (I) and the pharmaceutically acceptable salts thereof in the treatment or prevention of a disease or conditions which responds to aromatase inhibition, in particular a proliferative disease such as breast cancer or similar soft tissue endocrine-sensitive cancer, most preferably estrogen-dependent conditions like gynecomastia, mammary and endometrial tumors, endometrioisis and premature labor. The compounds are also useful for the treatment or prevention of locally advanced or metastatic breast cancer in postmenopausal women with hormone receptor positive or unknown.

The compounds of the formula (I) and the pharmaceutically acceptable salts thereof may also be administered in combination with one or more agents having anti-neoplastic actions, such as anti-oestrogenic activity as described for example for exemestane, toremifene, fulvestrant, tamoxifen; such as bone resorption inhibititory activity as described for example for pamidronate, zoledronic acid, such as alkylating activity as described for busulfan, temozolomide, melphalan, chlorambucil, mechlorethalamine, such as nucleotide base intercalating activity as described for example for adriamycin, daunorubicin, dactinomcyin, doxorubicin, epirubicin, idarubicin; such as anti-metabolite activity as described for example for cytarabine, fludarabine, cladrbine, mercaptopurine, thioguanine, capecitabine; such as anti-androgenic activity as described for example for abarelix, bicalutamide; such as androgenic activity as described for example for nilutamide, methyltestosterone; such as gonadotropin releasing hormone activity as described for example for leuprolide, triptorelin, goserelin; such as progestogenic activity as described for example for medroxyprogesterone, such as nucleoside analogue activity as described for example for gemcitarabine; such as topoisomerase I inhibitory activity as described for example for topotecan, irinotecan; such as kinase inhibitory activity as described for example for imatinib; such as growth factor inhibitory activity as described for example for gefitinib, trastuzumab; such as growth hormone activity as described for example for epoetin alfa, sargramostim, filgastrim, pegfilgastrim, oprelvekin, interferon alpha 2b; such as miscellaneous anti-tumor activity as described for example for pemetrexed, dacarbazine, procarbazine, oxaliplatin, asparaginase, pegaspargase, altetamine, gemtuzumab, vinorelbine, mitoxantrone, denileukin, rituximab, alitretinoin, arsenic trioxide, bortezomib, tretinoin, docetaxel; such as antiemetic activity as described for example for dolasetron, palonosetron, aprepitant, ganisetron, dronabinol, odansetron.

The compounds described in the present invention may be used as follows:

- As therapeutic combination in form of a preparation or a kit that is composed of individual components, including a herein described compound of the formula (I) and the pharmaceutically usable salts thereof and at least one medication with anti-neoplastic activity that can be administered either simultaneously or sequentially. The preparation or the kit may contain instructions of usage.

The dose may vary within wide limits and has of course to be adapted to the individual circumstances in each individual case. In general, for oral administration, a daily dose of about 0.3 mg to about 3 g, preferably about 1 mg to about 1 g, for example about 10 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

## EXAMPLES

The following examples illustrate the present invention. All temperatures are stated in degrees Celsius, pressures in mbar. Unless mentioned otherwise, the reactions take place at room temperature. The abbreviation "Rf = xx (A)" means for example that the Rf is found in solvent system A to have the value xx. The proportion of solvents to one another is always stated in fractions by volume. Chemical names of end products and intermediates were generated with the aid of the AutoNom 2000 (Automatic Nomenclature) program.

HPLC gradients on Hypersil BDS C-18 (5 μm); column: 4 × 125 mm:

(I) 90% water */10% acetonitrile * to 0% water */100% acetonitrile * in 5 minutes + 2.5 minutes (1.5 ml/min)
(II) 99% water */1% acetonitrile * to 0% water */100% acetonitrile * in 10 minutes + 2 minutes (1.5ml/min)

HPLC gradients on Synergi 4μm POLAR-RP 80A; column 4.60 x 100 mm:

(III) 90% water */10% acetonitrile * to 0% water */100% acetonitrile * in 5 minutes + 2.5 minutes (1.5ml/min)

* contains 0.1% trifluoroacetic acid

The abbreviations used are as follows:

Rf ratio of distance travelled by a substance to distance of the eluent from the starting point in thin-layer chromatography

Rt retention time of a substance in HPLC (in minutes)

m.p. melting point (temperature)

| | | |
|---|---|---|
| 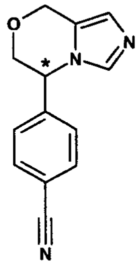 1 | 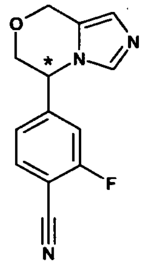 2 | 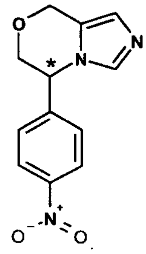 3 |
| 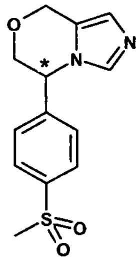 4 | 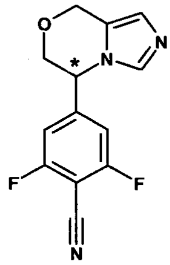 5 | 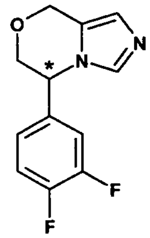 6 |

| | | |
|---|---|---|
| 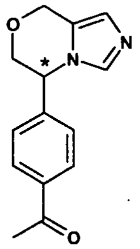 7 | 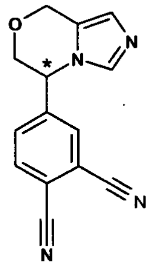 8 | |

Example 1

4-(5,6-Dihydro-8H-imidazo[5,1-c][1,4]oxazin-5-yl)benzonitrile

A solution of 1.00 mmol of 1-(4-cyanophenyl)-2-(1-trityl-1H-imidazol-4-ylmethoxy)ethyl methanesulphonate in 5 ml of N,N-dimethylformamide is mixed with 2.50 mmol of caesium carbonate and heated at 80°C for 6 hours. The reaction mixture is cooled to room temperature, diluted with water and extracted with ethyl acetate (2x). The combined organic phases are dried with sodium sulphate and evaporated. The title compound is obtained from the residue as yellowish crystals by flash chromatography ($SiO_2$ 60F). Rf = 0.61 (dichloromethane-methanol-25% aqueous ammonia solution 200:10:1); Rt = 3.54 (gradient II).

The starting materials are prepared as follows:

a) 1-(4-Cyanophenyl)-2-(1-trityl-1H-imidazol-4-ylmethoxy)ethyl methanesulphonate 4 mmol of triethylamine and 2.00 mmol of methanesulphonyl chloride are added to a solution of 1.00 mmol of 4-[1-hydroxy-2-(1-trityl-1H-imidazol-4-ylmethoxy)ethyl]benzonitrile in 10 ml of dichloromethane at 0°C. The reaction mixture is stirred at 0°C for 1 hour, diluted with dichloromethane, washed with 1 N HCl, dried with sodium sulphate and evaporated. The crude title compound is used without further purification in the next stage. Rf = 0.43 (dichloromethane-methanol 95:5); Rt = 4.46 (gradient I).

b1) 4-[1-Hydroxy-2-(1-trityl-1H-imidazol-4-ylmethoxy)ethyl]benzonitrile Sodium borohydride is added in portions to a solution of 1 mmol of 4-[2-(1-trityl-1H-imidazol-4-ylmethoxy)acetyl]benzonitrile in 12 ml of ethanol at 0°C. The reaction solution is stirred at room temperature for 12 hours, then poured into ice-water and stirred for 15 minutes. The mixture is adjusted to pH 5 by adding glacial acetic acid and extracted with tert-butyl methyl ether (2x). The combined organic phases are washed with water and brine, dried over sodium sulphate and evaporated. The title compound is identified from the residue on the basis of the Rf by flash chromatography ($SiO_2$ 60F).

c1) 4-[2-(1-Trityl-1H-imidazol-4-ylmethoxy)acetyl]benzonitrile A solution of 14 mmol of 4-iodobenzonitrile [3058-39-7] in 20 ml of tetrahydrofuran is cooled to -30°C, and 14.80 mmol of i-propylmagnesium chloride (2M in tetrahydrofuran) are added. The mixture is stirred at -30°C for 60 minutes and a solution, precooled to -30°C, of 10.0 mmol of N-methoxy-N-methyl-2-(1-trityl-1H-imidazol-4-ylmethoxy)acetamide in 30 ml of tetrahydrofuran is added. The mixture is stirred at -30°C for 30 minutes, and then the reaction mixture is warmed to room temperature and quenched with saturated aqueous ammonium chloride solution. The phases are separated, and the aqueous phase is extracted with ethyl acetate (3x). The combined organic phases are washed with brine, dried with magnesium sulphate and evaporated. The title compound is identified from the residue on the basis of the Rf by flash chromatography ($SiO_2$ 60F).

d1) N-Methoxy-N-methyl-2-(1-trityl-1H-imidazol-4-ylmethoxy)acetamide A solution of 4.03 mmol of (1-trityl-1H-imidazol-4-ylmethoxy)acetic acid and 4.44 mmol of N,O-dimethylhydroxylamine hydrochloride in 100 ml of dichloromethane is mixed with 20.2 mmol of triethylamine and 4.44 mmol of propanephosphonic acid cyclic anhydride [68957-94-8] (50% in ethyl acetate). The reaction mixture is stirred at room temperature for 3 hours and diluted with dichloromethane. The phases are separated and the organic phase is washed with 1M HCl and brine, dried with sodium sulphate and evaporated. The title compound is obtained as a pale yellowish solid from the residue by flash chromatography ($SiO_2$ 60F). Rf = 0.45 (dichloromethane-methanol 95:5); Rt = 4.11 (gradient I).

e1) (1-Trityl-1H-imidazol-4-ylmethoxy)acetic acid A mixture of 1.0 mmol of ethyl (1-trityl-1H-imidazol-4-ylmethoxy) acetate in 16 ml of tetrahydrofuran and 16 ml of 2N NaOH is stirred under reflux for 18 hours. The reaction mixture is cooled and the tetrahydrofuran is distilled off. 20 ml of 2N HCl are added to the aqueous residue, and the resulting suspension is diluted with tert-butyl methyl ether. The solid is filtered off and the filter cake is washed with water and tert-butyl methyl ether and dried. The title compound is obtained as a yellowish solid. Rf = 0.02 (ethyl acetate-heptane 2:1); Rt = 3.86 (*gradient I).

f) Ethyl (1-trityl-1H-imidazol-4-ylmethoxy)acetate 58.0 mmol of sodium hydride (60% dispersion in paraffin) are added in portions to a solution of 30.0 mmol of (1-trityl-1H-imidazol-4-yl)methanol [33769-07-2] in 300 ml of N,N-dimethylformamide at 20°C. The mixture is stirred at 20°C for 1.5 hours. 50.0 mmol of ethyl bromoacetate [105-36-2] and 6.00 mmol of potassium iodide are added, and the mixture is stirred at room temperature for 16 hours. A further 58.0 mmol of sodium hydride and 50 mmol of ethyl bromoacetate are added and the mixture is stirred again for 16 hours. The reaction mixture is poured into water and extracted with tert-butyl methyl ether (2x). The combined organic phases are washed with water and brine, dried with magnesium sulphate and evaporated. The title compound is obtained as a brown oil from the residue by flash chromatography ($SiO_2$ 60F). Rf = 0.20 (ethyl acetate-heptane 2:1), Rt = 4.32 (gradient I).

Alternative syntheses for 4-[1-hydroxy-2-(1-trityl-1H-imidazol-4-ylmethoxy)ethyl]benzonitrile:

b2) 4-[1-Hydroxy-2-(1-trityl-1H-imidazol-4-ylmethoxy)ethyl]benzonitrile 1.5 mmol of tetrabutylammonium fluoride (1 M solution in tetrahydrofuran) are added to a solution of 1 mmol of 4-[1-(tert-butyldimethylsilanyloxy)-2-(1-trityl-1H-imidazol-4-ylmethoxy)ethyl]benzonitrile in 5 ml of tetrahydrofuran, and the solution is stirred at room temperature for 1 hour. The reaction solution is then diluted with water and extracted with tert-butyl methyl ether (2x). The combined organic phases are dried with sodium sulphate and evaporated. The title compound is identified from the residue on the basis of the Rf by flash chromatography ($SiO_2$ 60F).

c2) 4-[1-(tert-Butyldimethylsilanyloxy)-2-(1-trityl-1H-imidazol-4-ylmethoxy)ethyl]benzonitrile A solution of 1.27 mmol of titanium tetrachloride in 1.5 ml of dichloromethane is added to a solution of 2.61 mmol of trimethylsilyl trifluoromethanesulphonate in 1 ml of dichloromethane at 0°C. The mixture is stirred at room temperature for 4 hours and then cooled to 0°C. A solution of 0.83 mmol of 1-trityl-1H-imidazol-4-ylmethyl (tert-butyldimethylsilyloxy)(4-cyanophenyl)acetate and 4.17 mmol of triethylsilane in 2 ml of dichloromethane is added, and the reaction mixture is stirred at room temperature for 20 hours. The reaction mixture is poured into ice-water and extracted with ethyl acetate (2x). The combined organic phases are washed with water and brine, dried with sodium sulphate and

evaporated. The title compound is identified from the residue on the basis of the Rf by flash chromatography ($SiO_2$ 60F).

d2) 1-Trityl-1H-imidazol-4-ylmethyl (tert-butyldimethylsilanyloxy)(4-cyanophenyl)acetate 5.0 mmol of triethylamine and 1.0 mmol propanephosphonic acid cyclic anhydride [68957-94-8] (50% in ethyl acetate) are added to a solution of 1.0 mmol of (1-trityl-1H-imidazol-4-yl)methanol [33769-07-2] and 1.0 mmol of (tert-butyldimethylsilanyloxy)(4-cyanophenyl)acetic acid in 20 ml of dichloromethane. The reaction mixture is stirred at room temperature for 3 hours and diluted with dichloromethane. The phases are separated and the organic phase is washed with 1 M HCl and brine, dried with sodium sulphate and evaporated. The title compound is identified from the residue on the basis of the Rf by flash chromatography ($SiO_2$ 60F).

e2) tert-Butyldimethylsilanyloxy)(4-cyanophenyl)acetic acid A mixture of 1.0 mmol of methyl (tert-butyldimethylsilanyloxy)(4-cyanophenyl)acetate [435344-67-5] in 12 ml of tetrahydrofuran, 12 ml of methanol and 12 ml of water is mixed with 4 mmol of lithium hydroxide and stirred at 0°C for 2 hours. 20 ml of 2N HCl are added to the reaction mixture, which is extracted with tert-butyl methyl ether (3x). The combined organic phases are washed successively with water and brine, dried with sodium sulphate, filtered and evaporated, and the crude title compound is identified on the basis of the Rf. The crude title compound is used without further purification in the next stage.

b3) 4-[1-Hydroxy-2-(1-trityl-1H-imidazol-4-ylmethoxy)ethyl]benzonitrile 20.0 mmol of sodium hydride (60% dispersion in paraffin) are added to a solution of 20.0 mmol of (1-trityl-1H-imidazol-4-yl)methanol [33769-07-02] in 120 ml of absolute N,N-dimethylformamide under argon. The mixture is heated at 100°C for 1 hour and then cooled to 40°C. A solution of 20.0 mmol of 4-oxiranylbenzonitrile [52695-39-3] in 10 ml of absolute N,N-dimethylformamide is added dropwise at 35-40°C, and the reaction mixture is stirred at 40°C for 15 minutes. The reaction mixture is cooled to room temperature, poured into ice-water and extracted with ethyl acetate. The combined organic phases are washed with water and brine, dried over sodium sulphate and evaporated. The title compound is obtained as a white solid from the residue by flash chromatography ($SiO_2$ 60F). Rf = 0.29 (dichloromethane-methanol 95:5); Rt = 4.26 (gradient I).
The racemic compound is fractionated into the enantiomers by chiral preparative HPLC to afford the title compound. The title compound is isolated as the enantiomer which elutes second. Rt * = 13.55 min.

* HPLC method:

Column: 250 × 50 mm CHIRALPAK® AD 20 μm
Mobile phase: $CO_2$/methanol 80:20
Flow rate: 240 ml/min
Detection: UV 250 nm
Temperature: 25°C
Pressure: 150 bar

The following compounds are prepared in analogy to the process described in example 1:

2 4-(5,6-Dihydro-8H-imidazo[5,1-c][1,4]oxazin-5-yl)-2-fluorobenzonitrile starting from 2-fluoro-4-iodobenzonitrile [137553-42-5].

3 5-(4-Nitrophenyl)-5,6-dihydro-8H-imidazo[5,1-c][1,4]oxazine starting from 1-iodo-4-nitrobenzene [636-98-6].

4 5-(4-Methanesulphonylphenyl)-5,6-dihydro-8H-imidazo[5,1-c][1,4]oxazine starting from 1-iodo-4-methanesulphonylbenzene [64984-08-3].

5 4-(5,6-Dihydro-8H-imidazo[5,1-c][1,4]oxazin-5-yl)-2,6-difluorobenzonitrile starting from 2,6-difluoro-4-iodobenzonitrile [14743-50-3].

6 5-(3,4-Difluorophenyl)-5,6-dihydro-8H-imidazo[5,1-c][1,4]oxazine starting from 2-(3,4-difluorophenyl)oxirane [111991-13-0]. Rf = 0.31 (dichloromethane-methanol 95:5); Rt = 4.20 (gradient II).

8 4-(5,6-Dihydro-8H-imidazo[5,1-c][1,4]oxazin-5-yl)-phthalonitrile starting from 4-iodo-phthalonitrile [69518-17-8].

Example 7

1-[4-5,6-Dihydro-8H-imidazo[5,1-c][1,4]oxazin-5-yl)phenyl]ethanone

0.47 mmol of methylmagnesium bromide solution (3M in diethyl ether) is added to a suspension of 0.47 mmol of 4-(5,6-dihydro-8H-imidazo[5,1-c][1,4]oxazin-5-yl)benzonitrile (Example 1) in 5 ml of absolute toluene. The reaction mixture is heated to reflux for 16 hours, cooled and mixed with dilute aqueous sodium bicarbonate solution. The mixture is extracted with ethyl acetate-dichloromethane 4:1, and the combined organic phases are washed with brine, dried with sodium sulphate and evaporated. The title compound is obtained as a slightly whitish solid from the residue by flash chromatography ($SiO_2$ 60F). Rf = 0.34 (dichloromethane-methanol 95:5); Rt = 3.54 (gradient II). The racemic compound is fractionated into the enantiomers by chiral preparative HPLC to afford the title compound.

## Claims

1. A compound of the general formula

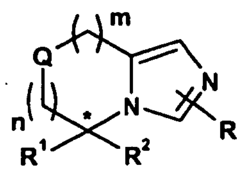

(I),

in which

R is deuterium, halogen or hydrogen;
R' is aryl-$C_0$-$C_4$-alkyl or heterocyclyl-$C_0$-$C_4$-alkyl, which radicals may be substituted by 1-4 $C_1$-$C_8$ alkoxy, $C_1$-$C_8$ alkoxycarbonyl, $C_1$-$C_8$ alkyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, oxide, oxo, tri-$C_1$-$C_4$-alkylsilyl, trifluoromethoxy or trifluoromethyl;
$R^2$ is

a) deuterium, halogen, hydroxy, cyano or hydrogen; or
b) $C_2$-$C_8$ alkenyl, $C_2$-$C_8$ alkynyl, $C_1$-$C_8$ alkoxy, $C_1$-$C_4$ alkoxycarbonyl-$C_1$-$C_4$ alkyl, $C_1$-$C_8$ alkyl, $C_0$-$C_4$ alkylcarbonyl, aryl-$C_0$-$C_4$ alkyl, carboxy-$C_1$-$C_4$ alkyl, $C_3$-$C_8$ cycloalkyl or heterocyclyl-$C_0$-$C_4$ alkyl, which radicals may be substituted by 1-4 $C_1$-$C_8$ alkoxy, $C_1$-$C_8$ alkoxycarbonyl, $C_1$-$C_8$ alkyl, $C_0$-$C_8$ alkylcarbonyl, $C_1$-$C_8$ alkylsulphonyl, optionally substituted aryl, aryl-$C_0$-$C_4$ alkoxycarbonyl, cyano, halogen, optionally substituted heterocyclyl, hydroxy, nitro, oxide, oxo, tri-$C_1$-$C_4$ alkylsilyl, trifluoromethoxy or trifluoromethyl;

Q is oxygen or sulphur;
m is a number 0, 1 or 2;
n is a number 0, 1 or 2; and
* designates an asymmetric carbon atom; and

where
m and n are not simultaneously 0;
or a salt, preferably a pharmaceutically acceptable salt, thereof
and which compound shows an aromatase inhibitory activity at least 10 times higher, but preferably 20 times higher, or more preferably 40 times higher, than the compound of the formula (I) with the opposite configuration around the asymmetric carbon atom labelled "*"

2. A compound according to Claim 1, where R is deuterium or hydrogen.

3. A compound according to Claim 1 or 2, where $R^1$ is optionally mono-, di- or tri-substituted phenyl or optionally mono-, di- or tri-substituted benzofuranyl, benzo[b]thiophenyl, benzoimidazolyl, benzo[d]isothiazolyl, benzo[d]isoxazolyl, benzo[b]thiophenyl, imidazolyl, indazolyl, oxazolyl, pyridyl, pyrrolyl, thiazolyl or thiophenyl.

4. A compound according to any one of Claims 1 to 3, where $R^2$ is $C_1$-$C_8$ alkoxy, hydroxy, $C_1$-$C_8$ alkyl, aryl-$C_0$-$C_4$ alkyl, deuterium, halogen, cyano or hydrogen.

5. A compound according to any one of Claims 1 to 4, where Q is oxygen.

6. The use of a compound of the general formula (I) or a pharmaceutically acceptable salt thereof according to any one of Claims 1 to 5 for the manufacture of a medicament.

7. Use of a compound of the general formula (I) or a pharmaceutically acceptable salt thereof according to any one of Claims 1 to 5, for producing a human medicament for the prevention, for delaying the progression or for the treatment of a disease or condition which responds to aromatase inhibition, in particular a proliferative disease.

8. Pharmaceutical product comprising a compound of the general formula (I) or a pharmaceutically acceptable salt thereof according to any one of Claims 1 to 5, and conventional excipients.

**Patentansprüche**

1. Eine Verbindung der allgemeinen Formel

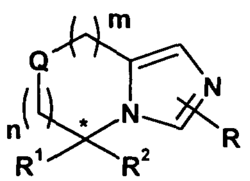

(I) ,

worin

R Deuterium, Halogen oder Wasserstoff bedeutet;
$R^1$ Aryl-$C_0$-$C_4$-alkyl oder Heterocyclyl-$C_0$-$C_4$-alkyl bedeutet, wobei diese Radikale substituiert sein können mit 1-4 $C_1$-$C_8$ Alkoxy, $C_1$-$C_8$ Alkoxycarbonyl, $C_1$-$C_8$ Alkyl, $C_0$-$C_8$ Alkylcarbonyl, $C_1$-$C_8$ Alkylsulphonyl, optional substituiertes Aryl, Aryl-$C_0$-$C_4$ Alkoxycarbonyl, Cyano, Halogen, optional substituiertes Heterocyclyl, Hydroxy, Nitro, Oxid, Oxo, Tri-$C_1$-$C_4$-alkylsilyl, Trifluoromethoxy oder Trifluoromethyl;
$R^2$

a) Deuterium, Halogen, Hydroxy, Cyano oder Wasserstoff bedeutet; oder
b) $C_2$-$C_8$ Alkenyl, $C_2$-$C_8$ Alkynyl, $C_1$-$C_8$ Alkoxy, $C_1$-$C_4$ Alkoxycarbonyl-$C_1$-$C_4$ Alkyl, $C_1$-$C_8$Alkyl, $C_0$-$C_4$ Alkylcarbonyl, Aryl-$C_0$-$C_4$ alkyl, Carboxy-$C_1$-$C_4$ alkyl, $C_3$-$C_8$ Cycloalkyl oder Heterocycyl-$C_0$-$C_4$ alkyl bedeutet, wobei diese Radikale substituiert sein können mit 1-4 $C_1$- $C_8$ Alkoxy, $C_1$-$C_8$ Alkoxycarbonyl, $C_1$-$C_8$ Alkyl, $C_0$-$C_8$ Alkylcarbonyl, $C_1$-$C_8$ Alkylsulphonyl, optional substituiertes Aryl, Aryl-$C_0$-$C_4$ Alkoxycarbonyl, Cyano, Halogen, optional substituiertes Heterocyclyl, Hydroxy, Nitro, Oxid, Oxo, Tri-$C_1$-$C_4$ Alkylsilyl, Trifluoromethoxy oder Trifluoromethyl;

Q Sauerstoff oder Schwefel bedeutet;
m eine Zahl 0, 1 oder 2 ist;
n eine Zahl 0, 1 oder 2 ist; und
* bezeichnet ein asymmetrisches Kohlenstoffatom; und

wobei
m und n nicht gleichzeitig 0 sind;
oder ein Salz davon, vorzugsweise ein pharmazeutisch akzeptables Salz,
und wobei diese Verbindung eine wenigstens 10 mal höhere Aromatase hemmende Wirkung aufweist, bevorzugt eine wenigstens 20 mal höhere, insbesondere bevorzugt eine wenigstens 40 mal höhere hemmende Wirkung aufweist, als eine Verbindung der Formel (I) mit der entgegengesetzten Konfiguration als das mit "*" **gekennzeichnet**e asymmetrische Kohlenstoffatom.

2. Eine Verbindung gemäss Anspruch 1, wobei R Deuterium oder Wasserstoff bedeutet.

3. Eine Verbindung gemäss Anspruch 1 oder 2, wobei $R^1$ optional substituiertes mono-, di-oder tri-substituiertes Phenyl oder optional mono-, di- oder tri-substituiertes Benzofuranyl, Benzo[b]thiophenyl, Benzoimidazolyl, Benzo[d]isothiazolyl, Benzo[d]isoxazolyl, Benzo[b]thiophenyl, Imidazolyl, Indazolyl, Oxazolyl, Pyridyl, Pyrrolyl, Thiazolyl oder Thiophenyl bedeutet.

4. Eine Verbindung gemäss einem der Ansprüche 1 bis 3, wobei $R^2$ $C_1$-$C_8$ Alkoxy, Hydroxy, $C_1$-$C_8$ Alkyl, Aryl-$C_0$-$C_4$ alkyl, Deuterium, Halogen, Cyano oder Wasserstoff bedeutet.

5. Eine Verbindung gemäss einem der Ansprüche 1 bis 4, wobei Q Sauerstoff bedeutet.

6. Die Verwendung einer Verbindung der allgemeinen Formel (I) oder eines pharmazeutisch akzeptablen Salzes davon gemäss einem der Ansprüche 1 bis 5, zur Herstellung eines Medikamentes.

7. Die Verwendung einer Verbindung der allgemeinen Formel (I) oder eines pharmazeutisch akzeptablen Salzes davon gemäss einem der Ansprüche 1 bis 5, zur Herstellung eines Humanmedikamentes zur Prävention, Verzögerung der Entwicklung oder zur Behandlung einer Krankheit oder Zustandes, welche/r auf eine Aromatase Hemmung anspricht, insbesondere eine proliferative Krankheit.

8. Ein pharmazeutisches Produkt umfassend eine Verbindung der allgemeinen Formel (I) oder eines pharmazeutisch akzeptablen Salzes davon gemäss einem der Ansprüche 1 bis 5, und herkömmlichen Arzneistoffträgern.

## Revendications

1. Composé de formule générale

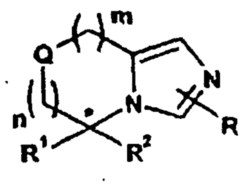

(I),

dans laquelle

R est deutérium, halogène ou hydrogène ;
$R^1$ est aryl-$C_0$-$C_4$-alkyle ou hétérocyclyl-$C_0$-$C_4$-alkyle, lesquels radicaux peuvent être substitués par 1-4 $C_1$-$C_8$ alcoxy, $C_1$-$C_8$ alcoxycarbonyle, $C_1$-$C_8$ alkyle, $C_0$-$C_8$ alkylcarbonyle, $C_1$-$C_8$ alkylsulfonyle, aryle éventuellement substitué, aryl-$C_0$-$C_4$ alcoxycarbonyle, cyano, halogène, hétérocyclyle éventuellement substitué, hydroxy, nitro, oxyde, oxo, tri-$C_1$-$C_4$-alkylsilyle, trifluorométhoxy ou trifluorométhyle ;
$R^2$ est

a) deutérium, halogène, hydroxy, cyano ou hydrogène ; ou
b) $C_2$-$C_8$ alcényle, $C_2$-$C_8$ alcynyle, $C_1$-$C_8$ alcoxy, $C_1$-$C_4$ alcoxycarbonyl- $C_1$-$C_4$ alkyle, $C_1$-$C_8$ alkyle, $C_0$-$C_4$ alkylcarbonyle, aryl-$C_0$-$C_4$ alkyle, carboxy-$C_1$-$C_4$ alkyle, $C_3$-$C_8$ cycloalkyle ou hétérocyclyl-$C_0$-$C_4$ alkyle, lesquels radicaux peuvent être substitués par 1-4 $C_1$-$C_8$ alcoxy, $C_1$-$C_8$ alcoxycarbonyle, $C_1$-$C_8$ alkyle, $C_0$-$C_8$ alkylcarbonyle, $C_1$-$C_8$ alkylsulfonyle, aryle éventuellement substitué, aryl-$C_0$-$C_4$ alcoxycarbonyle, cyano, halogène, hétérocyclyle éventuellement substitué, hydroxy, nitro, oxyde, oxo, tri-$C_1$-$C_4$ alkylsilyle, trifluorométhoxy ou trifluorométhyle ;

Q est oxygène ou soufre ;
m est un nombre 0, 1 ou 2 ;
n est un nombre 0, 1 ou 2 ; et
* désigne un atome de carbone asymétrique ; et

où
m et n ne sont pas simultanément 0 ;
ou un sel, de préférence un sel pharmaceutiquement acceptable, de celui-ci et lequel composé présente une activité inhibitrice d'aromatase au moins 10 fois plus élevée, mais de préférence 20 fois plus élevée, ou plus préférablement 40 fois plus élevée, que le composé de formule (I) avec la configuration inverse autour de l'atome de carbone asymétrique marqué "*".

2. Composé selon la revendication 1, **caractérisé en ce que** R est deutérium ou hydrogène.

3. Composé selon la revendication 1 ou 2, **caractérisé en ce que** $R^1$ est phényle éventuellement mono-, di- ou tri-substitué ou benzofuranyle, benzo[b]thiophényle, benzoimidazolyle, benzo[d]isothiazolyle, benzo[d]isoxazolyle, benzo[b]thiophényle, imidazolyle, indazolyle, oxazolyle, pyridyle, pyrrolyle, thiazolyle ou thiophényle éventuellement mono-, di- ou tri-substitué.

4. Composé selon l'une quelconque des revendications 1 à 3, **caractérisé en ce que** $R^2$ est $C_1$-$C_8$ alcoxy, hydroxy, $C_1$-$C_8$ alkyle, aryl-$C_0$-$C_4$ alkyle, deutérium, halogène, cyano ou hydrogène.

5. Composé selon l'une quelconque des revendications 1 à 4, **caractérisé en ce que** Q est oxygène.

6. Utilisation d'un composé de formule générale (I) ou d'un sel pharmaceutiquement acceptable de celui-ci selon une quelconque des revendications 1 à 5 pour la fabrication d'un médicament.

7. Utilisation d'un composé de formule générale (I) ou d'un sel pharmaceutiquement acceptable de celui-ci selon l'une quelconque des revendications 1 à 5 pour la production d'un médicament humain destiné à prévenir, retarder la progression ou traiter une maladie ou une affection qui réagit à l'inhibition d'aromatase, en particulier une maladie proliférative.

8. Produit pharmaceutique comprenant un composé de formule générale (I) ou un sel pharmaceutiquement acceptable de celui-ci selon l'une quelconque des revendications 1 à 5, et des excipients classiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Proc. Soc. Exp. Biol. Med.,* 1979, vol. 160, 296-301